# EUROPEAN PATENT APPLICATION

(11) **EP 4 722 333 A1**
(43) Date of publication of application: **08.04.2026**
(21) Application number: 24823480.9
(22) Date of filing: 14.06.2024
(51) Int. Cl.: C12M 1/00

(54) **DATA PROCESSING DEVICE AND DATA PROCESSING METHOD**

(30) Priority: 16.06.2023 JP 2023098864
(71) Applicant: Terumo Kabushiki Kaisha, Tokyo 151-0072 (JP)
(72) Inventor: YAMAZAKI, Nozomu, Ashigarakami-gun, Kanagawa 259-0151 (JP); IGARASHI, Masatsugu, Ashigarakami-gun, Kanagawa 259-0151 (JP)
(74) Representative: TBK
(86) International application number: PCT/JP2024/021740
(87) International publication number: WO 2024/257867

(57) **Abstract**

A data processing apparatus (14) includes an acquisition unit (34) that acquires first cell count data (42) and second cell count data (24), a calculation unit (36) that calculates a rate ratio that is a ratio between a first change rate of the first cell count data (42) in a first predetermined period and a second change rate of the second cell count data (24) in a second predetermined period corresponding to the first predetermined period, and a prediction data generation unit (38) that generates prediction data (48) indicating a change in the number of cells after the present time under the second culture condition on the basis of first partial data (42a) that is a part after the first predetermined period of the first cell count data (42) and the rate ratio.

## Description

### Technical Field

The present invention relates to a data processing apparatus and a data processing method of processing data obtained by culturing cells.

### Background Art

For example, JP 2020-171241 A discloses a cell culture apparatus. A user sets a culture condition of a cell culture apparatus so that a desired number of cells can be recovered in a desired culture period. The cell culture apparatus cultures cells under a set culture condition.

### Citation List

### Patent Literature

Patent Literature 1: JP 2020-171241 A

### Summary of Invention

### Technical Problem

In cell culture using a hollow fiber membrane, it is difficult to directly observe the inside of the hollow fiber during cell culture. Therefore, it is required to predict transition of cell culture during culture.

### Solution to Problem

An object of the present invention is to solve the above-described problem.
(1) A data processing apparatus according to a first invention is a data processing apparatus that performs data processing of cell culture performed by a cell culture apparatus including a hollow fiber membrane, the data processing apparatus including an acquisition unit that acquires first cell count data indicating a change in the number of cells from a start to an end of culture under a first culture condition and second cell count data indicating a change in the number of cells from a start of culture to a present time under a second culture condition, a calculation unit that calculates a rate ratio that is a ratio between a first change rate that is a change rate of the first cell count data in a first predetermined period and a second change rate that is a change rate of the second cell count data in a second predetermined period corresponding to the first predetermined period, and a prediction data generation unit that generates prediction data indicating a change in the number of cells after the present time under the second culture condition on the basis of first partial data that is a part after the first predetermined period of the first cell count data and the rate ratio.
   According to the configuration of the above-described item (1), since the prediction data reflecting the second cell count data, which is the data during culture, is generated, the transition of culture can be predicted during culture. As a result, the user can change an initial culture plan as needed during culture. Therefore, the cells can be suitably cultured.
(2) The data processing apparatus according to the above-described item (1) may further include a display control unit that performs control to display, on a display unit, the number of cells calculated from third cell count data that is data obtained by combining the prediction data with the second cell count data.
   According to the configuration of the above-described item (2), the user can grasp the culture period during which a desired number of cells can be recovered according to the number of cells displayed on the display unit.
(3) In the data processing apparatus according to the above-described item (1) or (2), the prediction data generation unit may generate the prediction data in a case where a deviation amount of the second change rate from the first change rate becomes a predetermined amount or larger.
   According to the configuration of the above-described item (3), the prediction data can be created only in a case where the difference between the initial culture plan and the actual culture situation becomes large.
(4) In the data processing apparatus according to any one of the above-described items (1) to (3), the first cell count data may be data obtained by cell culture performed in the past or data obtained by a simulation of cell culture.
(5) In the data processing apparatus according to any one of the above-described items (1) to (4), the acquisition unit may acquire the first cell count data on the basis of a plurality of pieces of cell count data.
(6) A data processing method according to a second invention includes acquiring first cell count data indicating a change in the number of cells from a start to an end of culture under a first culture condition and second cell count data indicating a change in the number of cells from a start of culture to a present time under a second culture condition, calculating a rate ratio that is a ratio between a first change rate that is a change rate of the first cell count data in a first predetermined period and a second change rate that is a change rate of the second cell count data in a second predetermined period corresponding to the first predetermined period, and generating prediction data indicating a change in the number of cells after the present time under the second culture condition on the basis of first partial data that is a part after the first predetermined period of the first cell count data and the rate ratio.

According to the configuration of the above-described item (6), as in the configuration of the above-described item (1), since the prediction data reflecting the second cell count data, which is the data during culture, is generated, the transition of culture can be predicted during culture. As a result, the user can change an initial culture plan as needed during culture. Therefore, the cells can be suitably cultured.

According to the present invention, it is possible to predict the transition of culture during culture of cells.

### Brief Description of Drawings

Fig. 1 is a block diagram of a cell culture system.
Fig. 2 is a circuit diagram of a cell culture circuit.
Fig. 3 is a diagram illustrating an operation of a cell culture apparatus at the time of cell culture in a first culture form.
Fig. 4 is a diagram illustrating an operation of the cell culture apparatus at the time of cell culture in a second culture form.
Fig. 5 is a diagram illustrating an operation of the cell culture apparatus at the time of cell culture in a third culture form.
Fig. 6 is a diagram illustrating an operation of the cell culture apparatus at the time of cell culture in a fourth culture form.
Fig. 7 is a flowchart of data display processing.
Fig. 8A is a graph illustrating first cell count data and second cell count data. Fig. 8B is a graph illustrating the first cell count data and third cell count data.
Fig. 9 is a diagram illustrating transition data displayed on a display unit.
Fig. 10 is a table illustrating evaluation results of an example.
Fig. 11 is a table illustrating a culture condition of each of three specimens.
Fig. 12 is a graph illustrating cell count data (glucose) obtained by simulating a specimen 2 (low) using a specimen 1 (middle) of adherent cells as reference data.
Fig. 13 is a graph illustrating cell count data (lactic acid) obtained by simulating the specimen 2 (low) using the specimen 1 (middle) of the adherent cells as the reference data.
Fig. 14 is a graph illustrating cell count data (oxygen) obtained by simulating the specimen 2 (low) using the specimen 1 (middle) of the adherent cells as the reference data.
Fig. 15 is a graph illustrating cell count data (carbon dioxide) obtained by simulating the specimen 2 (low) using the specimen 1 (middle) of the adherent cells as the reference data.
Fig. 16 is a graph illustrating cell count data (glucose) obtained by simulating the specimen 3 (high) using the specimen 1 (middle) of the adherent cells as the reference data.
Fig. 17 is a graph illustrating cell count data (lactic acid) obtained by simulating the specimen 3 (high) using the specimen 1 (middle) of the adherent cells as the reference data.
Fig. 18 is a graph illustrating cell count data (oxygen) obtained by simulating the specimen 3 (high) using the specimen 1 (middle) of the adherent cells as the reference data.
Fig. 19 is a graph illustrating cell count data (carbon dioxide) obtained by simulating the specimen 3 (high) using the specimen 1 (middle) of the adherent cells as the reference data.
Fig. 20 is a table illustrating a culture conditions.
Fig. 21 is a table illustrating a culture conditions.
Fig. 22 is a table illustrating a culture condition.
Fig. 23 is a graph illustrating cell count data (glucose) obtained by simulating the specimen 2 (low) using the specimen 1 (middle) of the floating cells as the reference data.
Fig. 24 is a graph illustrating cell count data (lactic acid) obtained by simulating the specimen 2 (low) using the specimen 1 (middle) of the floating cells as the reference data.
Fig. 25 is a graph illustrating cell count data (oxygen) obtained by simulating the specimen 2 (low) using the specimen 1 (middle) of the floating cells as the reference data.
Fig. 26 is a graph illustrating cell count data (carbon dioxide) obtained by simulating the specimen 2 (low) using the specimen 1 (middle) of the floating cells as the reference data.
Fig. 27 is a graph illustrating cell count data (glucose) obtained by simulating the specimen 3 (high) using the specimen 1 (middle) of the floating cells as the reference data.
Fig. 28 is a graph illustrating cell count data (lactic acid) obtained by simulating the specimen 3 (high) using the specimen 1 (middle) of the floating cells as the reference data.
Fig. 29 is a graph illustrating cell count data (oxygen) obtained by simulating the specimen 3 (high) using the specimen 1 (middle) of the floating cells as the reference data.
Fig. 30 is a graph illustrating cell count data (carbon dioxide) obtained by simulating the specimen 3 (high) using the specimen 1 (middle) of the floating cells as the reference data.

### Description of Embodiments

Usually, before culturing cells, a user predicts transition of a change in the number of cells (metabolic state) in cell culture to be performed with reference to past data, and creates a culture plan on the basis of a prediction result. For example, the user creates a culture period in which a desired number of cells can be recovered, and cultures cells in a cell culture apparatus during the created culture period.

However, when cells are actually cultured, the transition of the change in the number of cells might deviate from the predicted transition. In a case where the transition of an actual change in the number of cells significantly falls below the predicted transition, a desired number of cells cannot be recovered in the initially created culture period. The embodiment described below makes it possible to predict the transition of the change in the number of cells using data of the actual change in the number of cells during the cell culture.

### [1 Configuration of Cell Culture System 10]

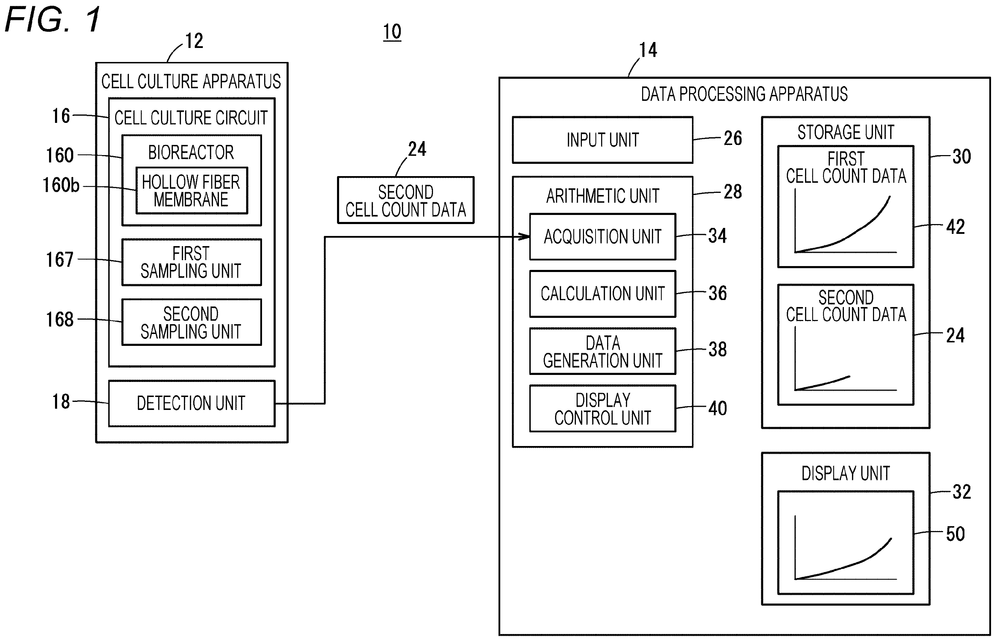

Fig. 1 is a block diagram of a cell culture system 10. The cell culture system 10 includes a cell culture apparatus 12 and a data processing apparatus 14. The data processing apparatus 14 predicts the transition of the change in the number of cells and displays the transition of the number of cells according to the prediction result. The cells used in the cell culture system 10 may be mammalian cells or cells derived from a mammal. The cells used in the cell culture system 10 may be adherent cells or floating cells. Examples of the cells include, for example, human embryonic kidney cells (HEK 293 cells), embryonic stem cells (ES cells), induced pluripotent stem cells (iPS cells), mesenchymal stem cells, fibroblast cells, endothelial cells, neural stem cells and the like. Examples of the floating cells include Jurkat cells (human cellular leukemia-derived cells), T cells, regulatory T cells, tumor-infiltrating lymphocytes, CAR-T cells, CD34 positive cells and the like. The cells used in the cell culture system 10 are not limited to those described above.

For cell culture, a basal medium and a complete medium are used. As the basal medium, for example, a minimum essential medium (MEM) is used. As the complete medium, a basal medium (for example, MEM) containing a nutrient component such as protein is used. As the protein, albumin, growth factors, cytokines and the like are used. For example, bovine serum containing albumin, growth factors and the like is added to the basal medium.

### [1-1 Cell Culture Apparatus 12]

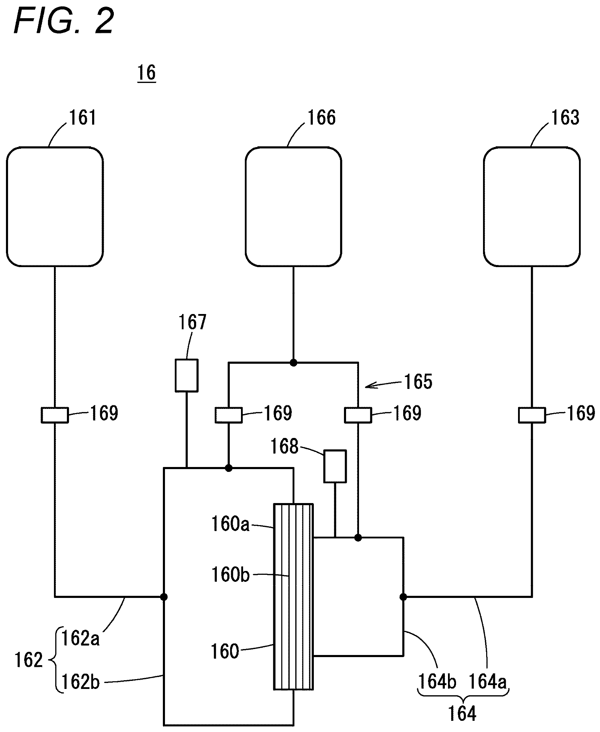

The cell culture apparatus 12 includes a cell culture circuit 16 and a detection unit 18. As illustrated in Fig. 2, the cell culture circuit 16 includes a bioreactor 160, a first supply unit 161, a first circuit 162, a second supply unit 163, a second circuit 164, a third circuit 165, a waste liquid storage unit 166, a first sampling unit 167, and a second sampling unit 168. The first sampling unit 167 and the second sampling unit 168 include various sensors (not illustrated) that detect concentrations of glucose, lactic acid, protein and the like. The cell culture circuit 16 includes various gas sensors that detect concentrations of gases (such as O₂ and CO₂) in the liquid. The cell culture circuit 16 includes a plurality of pumps (not illustrated) that applies a flow force to the liquid and a plurality of valves 169 that can open and close the flow path.

The bioreactor 160 includes a housing 160a and a plurality of hollow fiber membranes 160b. The housing 160a stores a plurality of hollow fiber membranes 160b. Each hollow fiber membrane 160b has a cylindrical shape. That is, each hollow fiber membrane 160b includes a pore (inner pore) penetrating from one end to the other end. The bioreactor 160 includes a flow path defined by an inner peripheral surface of the hollow fiber membrane 160b and a flow path defined by an inner wall surface of the housing 160a and an outer peripheral surface of the hollow fiber membrane 160b. Inside the housing 160a, a fluid can flow from the inside to the outside of the hollow fiber membrane 160b and from the outside to the inside of the hollow fiber membrane 160b.

The first supply unit 161 includes a bag storing a liquid such as a cell fluid, the complete medium, the basal medium, and a cleaning liquid. The first circuit 162 includes a first supply flow path 162a and a first circulation flow path 162b. The first supply flow path 162a connects the first supply unit 161 and the first circulation flow path 162b. The first circulation flow path 162b includes a flow path defined by the inner peripheral surface of each hollow fiber membrane 160b of the bioreactor 160. The liquid supplied from the first supply unit 161 to the first circulation flow path 162b via the first supply flow path 162a can circulate in the first circulation flow path 162b. The first sampling unit 167 samples the culture medium from the first circulation flow path 162b.

The second supply unit 163 includes a bag storing the liquid such as the basal medium and the cleaning liquid. The second circuit 164 includes a second supply flow path 164a and a second circulation flow path 164b. The second supply flow path 164a connects the second supply unit 163 and the second circulation flow path 164b. The second circulation flow path 164b includes the flow path defined by the inner wall surface of the housing 160a of the bioreactor 160 and the outer peripheral surface of the hollow fiber membrane 160b. The liquid supplied from the second supply unit 163 to the second circulation flow path 164b via the second supply flow path 164a can circulate in the second circulation flow path 164b. The second sampling unit 168 samples the culture medium from the second circulation flow path 164b.

The third circuit 165 connects the first circulation flow path 162b and the waste liquid storage unit 166, and connects the second circulation flow path 164b and the waste liquid storage unit 166. The waste liquid storage unit 166 includes a container for storing a waste liquid. The waste liquid discharged from the first circulation flow path 162b and the second circulation flow path 164b is recovered in the waste liquid storage unit 166.

One of the plurality of valves 169 is disposed in the first supply flow path 162a. One of the plurality of valves 169 is disposed in the second supply flow path 164a. One of the plurality of valves 169 is disposed in the flow path of the third circuit 165 connecting the first circulation flow path 162b and the waste liquid storage unit 166. One of the plurality of valves 169 is disposed in the flow path of the third circuit 165 connecting the second circulation flow path 164b and the waste liquid storage unit 166.

Returning to Fig. 1, the cell culture apparatus 12 will be continuously described. The detection unit 18 detects second cell count data 24 from the culture medium sampled by at least one of the first sampling unit 167 and the second sampling unit 168. The detection unit 18 transmits the second cell count data 24 to the data processing apparatus 14 every predetermined time. Note that, the second cell count data 24 is data indicating a change (increase or decrease) in the number of cells under a second culture condition. The second cell count data 24 is also data obtained by combining metabolic rates of all cells present in the bioreactor 160. Note that, the cell count data (second cell count data 24, and first cell count data 42 and third cell count data 44 to be described later) described in the present specification corresponds to, for example, data indicating the concentrations of glucose, lactic acid, O₂, CO₂ and the like in the culture medium. The second culture condition includes, for example, information regarding a substance contained in the culture medium, information regarding a gas supplied to the culture medium, information regarding the cell culture apparatus 12 (capacity information, operation information, culture form and the like), the number of culture days, seeding times, various temperatures and the like. The culture form will be described in the following [2] in detail.

### [1-2 Data Processing Apparatus 14]

The data processing apparatus 14 includes an input unit 26, an arithmetic unit 28, a storage unit 30, and a display unit 32. The data processing apparatus 14 may include, for example, a computer (personal computer, tablet terminal, smartphone and the like). Note that, in the data processing apparatus 14, the arithmetic unit 28 and the storage unit 30 may be provided in a server, and the display unit 32 may be provided in a terminal device.

The input unit 26 is a man-machine interface operated by a user. The input unit 26 may be, for example, a keyboard, a touch panel, a mouse and the like. The input unit 26 transmits the information input by the user to the arithmetic unit 28.

The arithmetic unit 28 can be configured by, for example, a processor such as a central processing unit (CPU) and a graphics processing unit (GPU). That is, the arithmetic unit 28 can be configured by a processing circuit.

The arithmetic unit 28 includes an acquisition unit 34, a calculation unit 36, a data generation unit 38 (prediction data generation unit), and a display control unit 40. Each of the acquisition unit 34, the calculation unit 36, the data generation unit 38, and the display control unit 40 can be implemented by executing a program stored in the storage unit 30 by the arithmetic unit 28.

Note that, at least a part of the acquisition unit 34, the calculation unit 36, the data generation unit 38, and the display control unit 40 may be implemented by an integrated circuit such as an application specific integrated circuit (ASIC) or a field-programmable gate array (FPGA). At least a part of the acquisition unit 34, the calculation unit 36, the data generation unit 38, and the display control unit 40 may be configured by an electronic circuit including a discrete device.

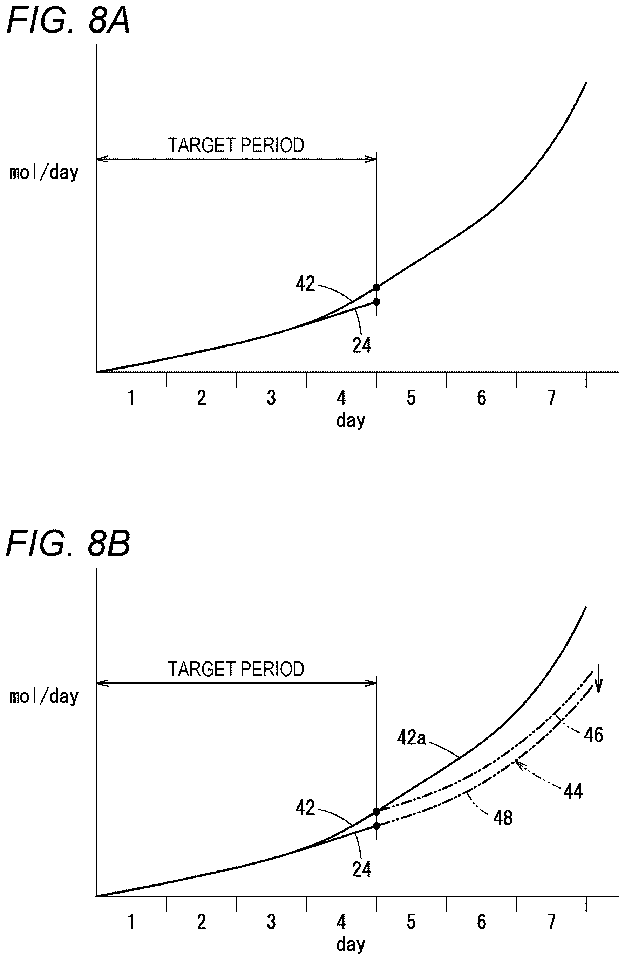

The acquisition unit 34 acquires data from a device outside the arithmetic unit 28 (cell culture apparatus 12, input unit 26, storage unit 30, external storage device and the like). The calculation unit 36 calculates a first change rate, a second change rate, and a rate ratio to be described later. The data generation unit 38 generates correction data 46 and prediction data 48 (Fig. 8B) on the basis of the first cell count data 42 and the rate ratio. Furthermore, the data generation unit 38 generates the third cell count data 44 on the basis of the second cell count data 24 and the prediction data 48. The display control unit 40 performs display control of the display unit 32.

The storage unit 30 can include a volatile memory not illustrated and a non-volatile memory not illustrated. Examples of the volatile memory may include a random access memory (RAM) and the like. The volatile memory is used as a working memory of the processor, and temporarily stores data and the like necessary for processing or arithmetic operation. Examples of the non-volatile memory include a read only memory (ROM) and a flash memory. The non-volatile memory is used as a memory for storage, and stores a program, a table, a map and the like. At least a part of the storage unit 30 may be provided in the processor, the integrated circuit and the like as described above.

The storage unit 30 stores the first cell count data 42 in advance before the cell culture apparatus 12 cultures the cells. The first cell count data 42 is data indicating a change (increase or decrease) in the number of cells under a first culture condition. The first cell count data 42 is data obtained by combining the metabolic rates of all cells present in the bioreactor 160. The first culture condition includes, for example, information regarding a substance contained in the culture medium, information regarding a gas supplied to the culture medium, information regarding the cell culture apparatus 12 (capacity information, operation information, culture form and the like), the number of culture days, seeding times, various temperatures and the like.

The first cell count data 42 may be data obtained by cell culture performed in the past. The first cell count data 42 may be data obtained by a simulation of cell culture. The first cell count data 42 may be stored not in the storage unit 30 but in the external storage device.

The display unit 32 may be a display, for example. The display unit 32 displays various display objects (for example, transition data 50) in response to the control of the display control unit 40.

### [2 Culture Form by Cell Culture Circuit 16]

In the cell culture circuit 16, a controller (not illustrated) can control a plurality of pumps (not illustrated) and a plurality of valves 169 to change a flow path through which the culture medium flows and a flow rate of the culture medium. As a result, the cell culture circuit 16 can implement a plurality of culture forms. Hereinafter, first to eighth culture forms will be described as an example of the culture form.

The first culture form and the second culture form are mainly executed from an initial stage to a middle stage of a cell culture process. The third culture form and the fourth culture form are mainly executed from the middle stage to a later stage of the cell culture process. The fifth culture form is appropriately executed during the culture period in order to collect cells.

### [2-1 First Culture Form]

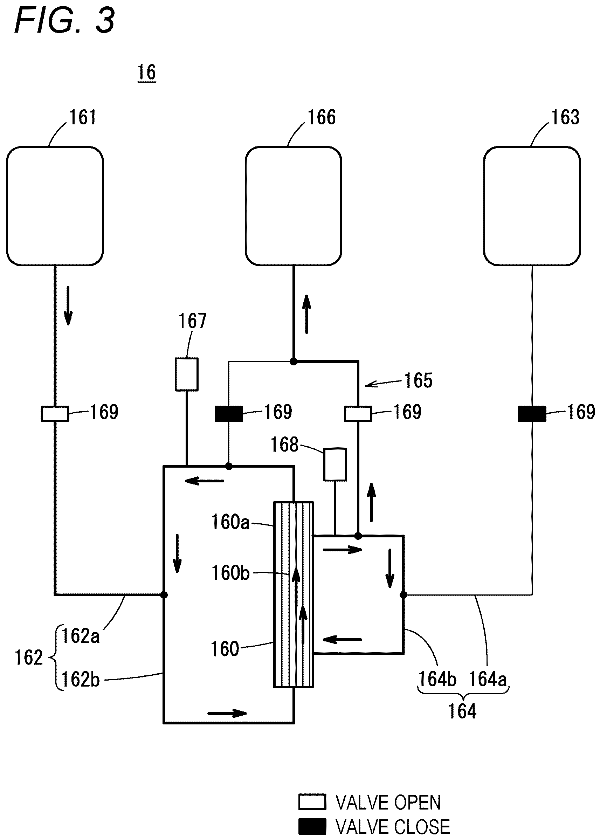

Fig. 3 is a diagram illustrating an operation of the cell culture apparatus 12 at the time of cell culture in the first culture form. The first culture form is as follows.
(a) The complete medium is supplied from the first supply unit 161 to the first circuit 162 (first circulation flow path 162b).
(b) The basal medium is not supplied from the second supply unit 163 to the second circuit 164 (second circulation flow path 164b).
(c) A part of the culture medium is discarded from the second circulation flow path 164b to the waste liquid storage unit 166 via the third circuit 165.

The first culture form is mainly executed in the cell culture process, but may be executed in a cell collection process. In a case where the first culture form is executed in the cell collection process, for example, the basal medium is supplied from both ends of the hollow fiber membrane 160b. As a result, the cells present in the first circulation flow path 162b can be collected in the hollow fiber membrane 160b.

### [2-2 Second Culture Form]

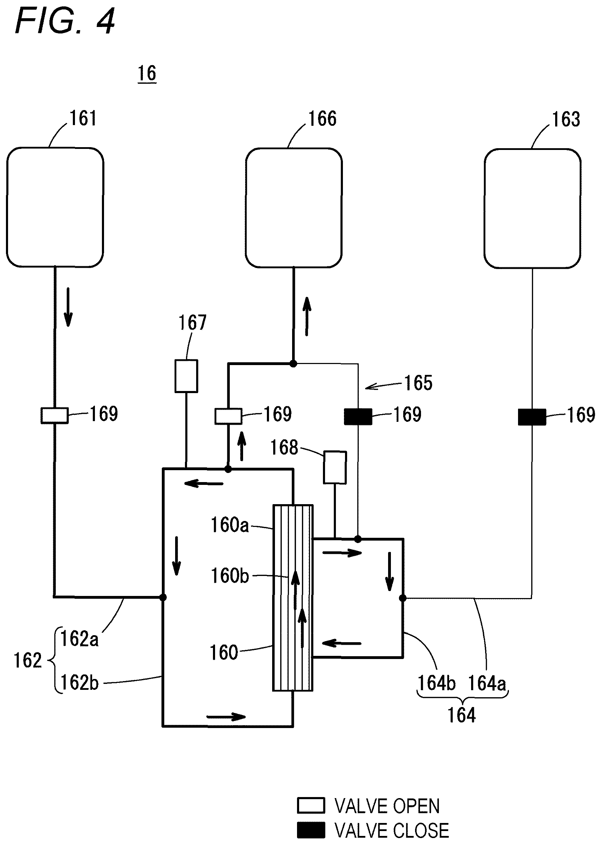

Fig. 4 is a diagram illustrating an operation of the cell culture apparatus 12 at the time of cell culture in the second culture form. The second culture form is as follows.
(a) The complete medium is supplied from the first supply unit 161 to the first circuit 162 (first circulation flow path 162b).
(b) The basal medium is not supplied from the second supply unit 163 to the second circuit 164 (second circulation flow path 164b).
(c) A part of the culture medium is discarded from the first circulation flow path 162b to the waste liquid storage unit 166 via the third circuit 165.

### [2-3 Third Culture Form]

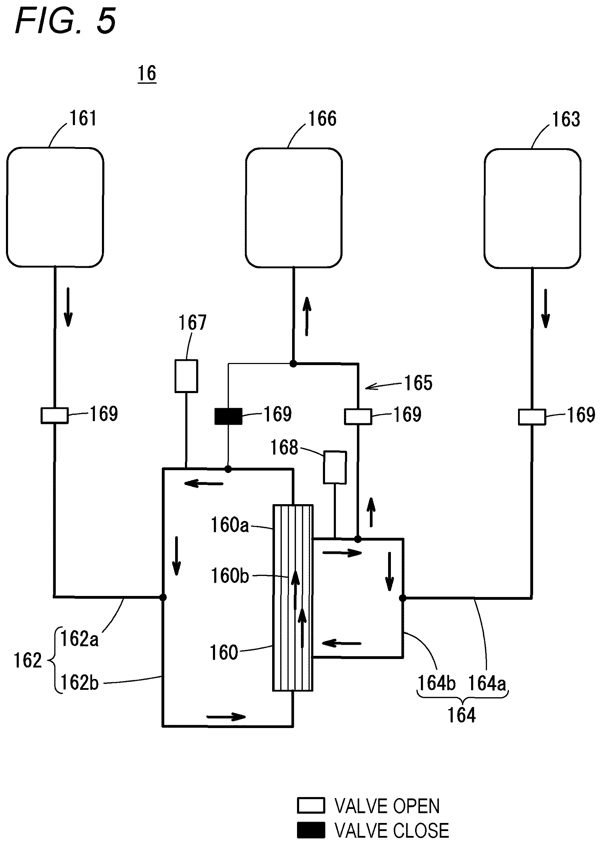

Fig. 5 is a diagram illustrating an operation of the cell culture apparatus 12 at the time of cell culture in the third culture form. The third culture form is as follows.
(a) The complete medium is supplied from the first supply unit 161 to the first circuit 162 (first circulation flow path 162b).
(b) The basal medium is supplied from the second supply unit 163 to the second circuit 164 (second circulation flow path 164b).
(c) A part of the culture medium is discarded from the second circulation flow path 164b to the waste liquid storage unit 166 via the third circuit 165.

### [2-4 Fourth Culture Form]

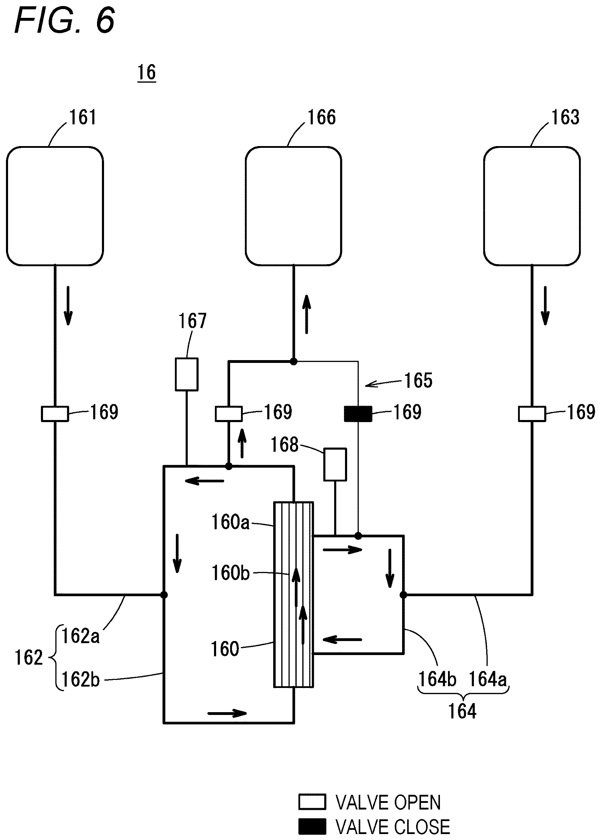

Fig. 6 is a diagram illustrating an operation of the cell culture apparatus 12 at the time of cell culture in the fourth culture form. The fourth culture form is as follows.
(a) The complete medium is supplied from the first supply unit 161 to the first circuit 162 (first circulation flow path 162b).
(b) The basal medium is supplied from the second supply unit 163 to the second circuit 164 (second circulation flow path 164b).
(c) A part of the culture medium is discarded from the first circulation flow path 162b to the waste liquid storage unit 166 via the third circuit 165.

### [2-5 Fifth Culture Form]

The fifth culture form is as follows. An operation of the cell culture apparatus 12 at the time of cell culture in the fifth culture form is the same as the operation of the cell culture apparatus 12 at the time of cell culture in the first culture form (Fig. 3) except for the following (a).
(a) The basal medium is supplied from the first supply unit 161 to the first circuit 162 (first circulation flow path 162b).
(b) The basal medium is not supplied from the second supply unit 163 to the second circuit 164 (second circulation flow path 164b).
(c) A part of the culture medium is discarded from the second circulation flow path 164b to the waste liquid storage unit 166 via the third circuit 165.

In the fifth culture form, the basal medium may be supplied from both ends of the hollow fiber membrane 160b as in the first culture form. As a result, the cells present in the first circulation flow path 162b can be collected in the hollow fiber membrane 160b. A step in the fifth culture form is defined as the cell collection process.

### [2-6 Sixth Culture Form]

The sixth culture form is as follows. An operation of the cell culture apparatus 12 at the time of cell culture in the sixth culture form is the same as the operation of the cell culture apparatus 12 at the time of cell culture in the second culture form (Fig. 4) except for the following (a).
(a) The basal medium is supplied from the first supply unit 161 to the first circuit 162 (first circulation flow path 162b).
(b) The basal medium is not supplied from the second supply unit 163 to the second circuit 164 (second circulation flow path 164b).
(c) A part of the culture medium is discarded from the first circulation flow path 162b to the waste liquid storage unit 166 via the third circuit 165.

### [2-7 Seventh Culture Form]

The seventh culture form is as follows. An operation of the cell culture apparatus 12 at the time of cell culture in the seventh culture form is the same as the operation of the cell culture apparatus 12 at the time of cell culture in the third culture form (Fig. 5) except for the following (a).
(a) The basal medium is supplied from the first supply unit 161 to the first circuit 162 (first circulation flow path 162b).
(b) The basal medium is supplied from the second supply unit 163 to the second circuit 164 (second circulation flow path 164b).
(c) A part of the culture medium is discarded from the second circulation flow path 164b to the waste liquid storage unit 166 via the third circuit 165.

### [2-8 Eighth Culture Form]

The eighth culture form is as follows. An operation of the cell culture apparatus 12 at the time of cell culture in the eighth culture form is the same as the operation of the cell culture apparatus 12 at the time of cell culture in the fourth culture form (Fig. 6) except for the following (a).
(a) The basal medium is supplied from the first supply unit 161 to the first circuit 162 (first circulation flow path 162b).
(b) The basal medium is supplied from the second supply unit 163 to the second circuit 164 (second circulation flow path 164b).
(c) A part of the culture medium is discarded from the first circulation flow path 162b to the waste liquid storage unit 166 via the third circuit 165.

The first culture form, the third culture form, the fifth culture form, and the seventh culture form are executed in a culture process of floating cells. The second culture form, the fourth culture form, the sixth culture form, and the eighth culture form are executed in a culture process of adherent cells. In each culture process of the floating cells and the adherent cells, one culture form among the plurality of culture forms described above may be executed, or a plurality of culture forms may be combined.

Each culture form is different in a supplied culture medium, a method of supplying the culture medium, a method of discarding the culture medium and the like. In a case of executing the generation of the third cell count data 44 (Fig. 8B) in any period, the data generation unit 38 can execute a simulation obtained by combining a plurality of culture forms.

### [3 Operation of Data Processing Apparatus 14]

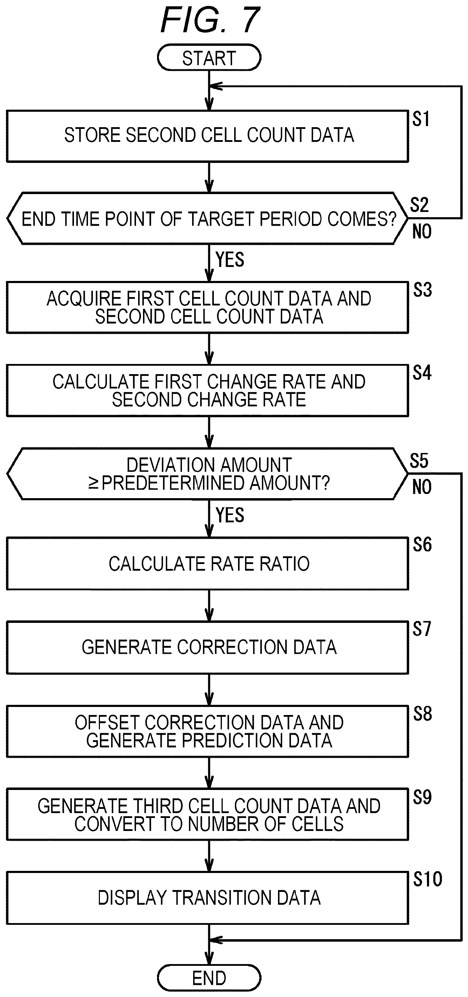

Fig. 7 is a flowchart of data display processing. The user causes the cell culture apparatus 12 to execute the cell culture and causes the data processing apparatus 14 to execute the data display processing.

Note that, the user operates the input unit 26 to designate a target period (first predetermined period and second predetermined period) before causing the data processing apparatus 14 to execute the data display processing. The target period is a period necessary for calculating the first change rate and the second change rate to be described later. The target period is a period selected by the user out of the cell culture period. As an example, in a case where the cell culture period lasts for seven days, the user can input "first day" as a start time point of the target period, and input "fourth day" as an end time point of the target period. Hereinafter, the target period will be described as "from first day to fourth day". The user can also input other periods. The first predetermined period that is the target period of the first cell count data 42 and the second predetermined period that is the target period of the second cell count data 24 are the same period. That is, the second predetermined period corresponds to the first predetermined period. After inputting the target period, the user operates the input unit 26 to cause the data processing apparatus 14 to execute data display processing.

At step S1, the acquisition unit 34 acquires the second cell count data 24 from the detection unit 18 of the cell culture apparatus 12. The storage unit 30 sequentially stores the acquired second cell count data 24.

At step S2, the calculation unit 36 determines whether the fourth day (the end point of the target period) comes. In a case where the fourth day comes (step S2: YES), the processing proceeds to step S3. In contrast, in a case where the fourth day does not come (step S2: NO), the processing returns to step S1.

When the processing proceeds from step S2 to step S3, the acquisition unit 34 acquires the first cell count data 42 and the second cell count data 24 from the storage unit 30. The first cell count data 42 acquired here is data indicating a change in the number of cells from the start of culture to the end of culture under the first culture condition. The second cell count data 24 acquired here is data indicating a change in the number of cells from the start of culture to a present time under the culture condition (second culture condition) of the cell culture currently being performed.

At step S4, the calculation unit 36 calculates the first change rate and the second change rate in the target period designated by the user. The change rate is a ratio between a designated target period (from first day to fourth day) and a change amount of the cell count data during the target period. For example, the calculation unit 36 calculates the first change rate by dividing the change amount of the first cell count data 42 in the target period (first predetermined period) of the first cell count data 42 by the target period. Similarly, the calculation unit 36 calculates the second change rate by dividing the change amount of the second cell count data 24 in the target period (second predetermined period) of the second cell count data 24 by the target period.

At step S5, the calculation unit 36 calculates a deviation amount of the second change rate from the first change rate. Furthermore, the calculation unit 36 compares the deviation amount with a predetermined amount. The predetermined amount is a threshold for determining whether to execute the processing at and after step S6. That is, the predetermined amount is a threshold for determining whether to display the transition data 50 on the display unit 32. The predetermined amount is stored in advance in the storage unit 30. In a case where the deviation amount is the predetermined amount or larger (step S5: YES), the processing proceeds to step S6. In this case, as illustrated in Fig. 8A, a deviation already occurs between the first cell count data 42 and the second cell count data 24 on the fourth day (the end point of the target period). In this case, there is a high possibility that the transition of the second cell count data 24 on and after the fourth day significantly deviates from the transition of the first cell count data 42. That is, at the end of the cell culture period, the number of cells is likely to be significantly less than the initially predicted number. In contrast, in a case where the deviation amount is less than the predetermined amount (step S5: NO), the data display processing ends. In this case, there is a high possibility that the transition of the second cell count data 24 after the fourth day is not significantly deviated from the transition of the first cell count data 42. That is, at the end of the cell culture period, the number of cells is likely to approximate the initially predicted number. Therefore, it is not necessary to display the transition data 50. Therefore, the data display processing ends.

In a case where the processing proceeds from step S5 to step S6, processing of predicting the transition of the second cell count data 24 after the fourth day is executed. For example, the calculation unit 36 calculates the rate ratio. The rate ratio is a ratio between the first change rate and the second change rate. For example, the calculation unit 36 calculates the rate ratio by dividing the second change rate by the first change rate.

At step S7, the data generation unit 38 generates the correction data 46 by correcting the first cell count data 42. As illustrated in Fig. 8B, for example, the data generation unit 38 generates the correction data 46 on the basis of first partial data 42a of the first cell count data 42 and the rate ratio. The first partial data 42a is partial data after the target period in the first cell count data 42. Here, the first partial data 42a is data from fifth day to seventh day of the first cell count data 42. The data generation unit 38 generates the correction data 46 by multiplying the first partial data 42a by the rate ratio.

At step S8, the data generation unit 38 brings the correction data 46 close to the second cell count data 24. Specifically, the data generation unit 38 offsets the correction data 46 by a difference between the first cell count data 42 and the second cell count data 24 on the fourth day (the end point of the target period). As a result, the data generation unit 38 generates the prediction data 48. As described above, the correction data 46 after offset is referred to as the prediction data 48. The prediction data 48 is data indicating a change in the number of cells after the present time under the second culture condition.

At step S9, the data generation unit 38 generates the third cell count data 44 (Fig. 8B) obtained by combining the prediction data 48 with the second cell count data 24. Furthermore, the data generation unit 38 converts the third cell count data 44 into the number of cells by a predetermined method.

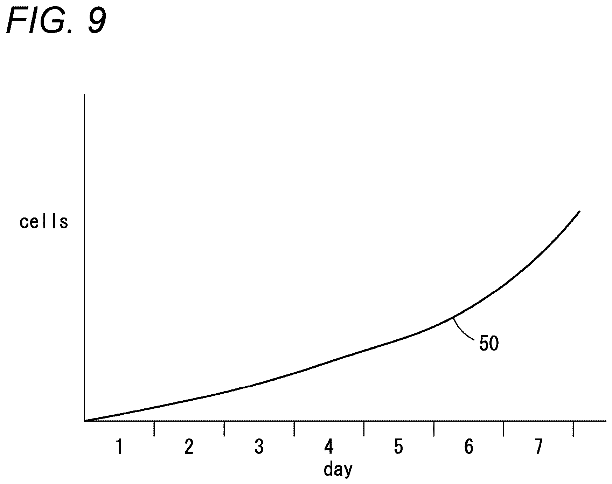

At step S10, the data generation unit 38 performs fitting using the data of the number of cells after the conversion, and generates the transition data 50 indicating the transition of the number of cells. The display control unit 40 performs control to display the transition data 50 on the display unit 32. Then, the display unit 32 displays the transition data 50 as illustrated in Fig. 9. Note that, the display control unit 40 may perform control to display the third cell count data 44 on the display unit 32 together with the transition data 50, or may perform control to display only the third cell count data 44 on the display unit 32.

### [4 Other Embodiments]

There may be a case where a plurality of pieces of cell count data is present before the cell culture. For example, in a case where there is the cell count data obtained in each of a plurality of times of cell culture in the past, or in a case where there is the cell count data obtained in each of a plurality of simulations, the acquisition unit 34 may acquire the first cell count data 42 on the basis of the plurality of pieces of cell count data. For example, the acquisition unit 34 may calculate an average of the plurality of pieces of cell count data to obtain the first cell count data 42.

The order of processing of multiplying the rate ratio at step S7 and offset processing at step S8 may be switched. That is, the data generation unit 38 may offset the first partial data 42a and use the first partial data 42a after the offset as the correction data. Furthermore, the data generation unit 38 may generate the prediction data 48 by multiplying the correction data by the rate ratio.

The display control unit 40 may allow the display unit 32 to display the transition data 50 and display an allowable range of the number of cells. In this case, the storage unit 30 stores the allowable range of the number of cells in advance.

The data generation unit 38 may calculate a timing (date and time) at which the number of cells designated by the user can be recovered on the basis of the transition data 50. In this case, the display control unit 40 may allow the display unit 32 to display the timing at which the number of cells designated by the user can be recovered.

In a case where the acquisition unit 34 acquires the second cell count data 24, the data generation unit 38 may calculate the allowable range of the cell count data. For example, the data generation unit 38 may calculate values of ±5%×day with respect to the second cell count data 24. The value of -5%×day with respect to the second cell count data 24 is a lower limit value of the allowable range. The value of +5%×day with respect to the second cell count data 24 is an upper limit value of the allowable range. The display control unit 40 may allow the display unit 32 to display the allowable range of the cell count data calculated by the data generation unit 38.

### [5 Effects of Each Embodiment]

According to each embodiment, since the prediction data 48 reflecting the second cell count data 24, which is data during culture, is generated, it is possible to predict the transition of culture during the culture. Since the prediction data 48 reflecting the second cell count data 24, which is data during culture, is generated, it is possible to predict the transition of culture during the culture. As a result, the user can change an initial culture plan as needed during culture. Therefore, the cells can be suitably cultured.

According to each embodiment, the user can grasp the culture period during which a desired number of cells can be recovered according to the number of cells displayed on the display unit 32.

According to each embodiment, the prediction data 48 can be created only in a case where a difference between the initial culture plan and an actual culture situation becomes large.

Note that, the present invention is not limited to the above-described disclosure, and various configurations can be adopted without departing from the gist of the present invention.

### [6 Example of Processing of Generating Third Cell Count Data 44]

The present inventors actually performed the processing of generating the third cell count data 44 on the basis of the above-described embodiment. Furthermore, the present inventors compared the generated third cell count data 44 with a measurement result measured in the actual cell culture to evaluate accuracy of the third cell count data 44. As a result, the third cell count data 44 was close to the actual measurement result. From this, the present inventors have concluded that the third cell count data 44 is appropriate. Hereinafter, an example of processing of generating the third cell count data 44 will be described.

The cell culture apparatus 12 used in a cell culture experiment is a Quantum cell expansion system (manufactured by Terumo BCT, Inc.) or a Quantum Flex cell expansion system (manufactured by Terumo BCT, Inc.). Hereinafter, the Quantum cell expansion system is referred to as a "first culture apparatus", and the Quantum Flex cell expansion system is referred to as a "second culture apparatus". A size of the bioreactor 160 is different between the first culture apparatus and the second culture apparatus. The size of the bioreactor 160 of the second culture apparatus is 1/10 the size of the bioreactor 160 of the first culture apparatus. In at least one of the first culture apparatus and the second culture apparatus, the size of the bioreactor 160 may be changeable.

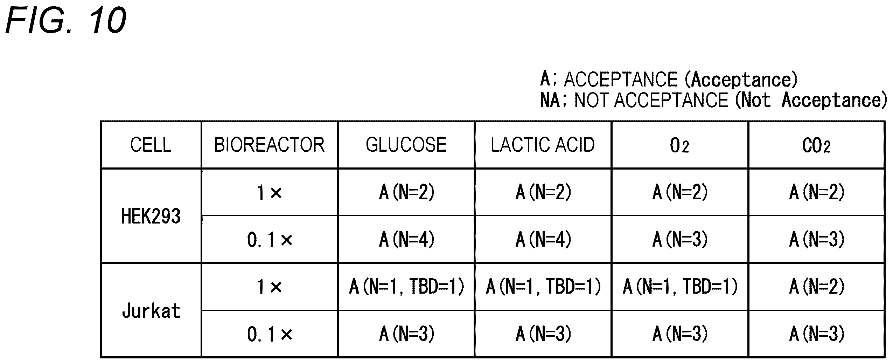

Fig. 10 is a table illustrating evaluation results of the example. In a column of "cell" in the table, cultured cells are entered. In a column of "bioreactor" in the table, the culture apparatus used is entered. In the column of "bioreactor", "1×" means that the first culture apparatus was used, and "0.1×" means that the second culture apparatus was used. In a column of "glucose" in the table, evaluation results are entered. In the column of "glucose", "A" means that the evaluation result is "acceptance". In the example, a simulation value falling within a range of ±5%×day with respect to a measured concentration was determined to be "acceptance". Note that, this time, there was no example evaluated as "not acceptance" (NA). "N" in parentheses means the number of pieces of evaluated processing (processing of generating the third cell count data 44). "TBD" in parentheses means the number of specimens whose simulation value did not fall within the range of ±5%×day with respect to the measured concentration on the final day of culture. Similarly to the column of "glucose", the evaluation results are also entered in each of columns of "lactic acid", "O₂", and "CO₂".

As illustrated in Fig. 10, regarding the simulation in which Jurkat cells are cultured by the first culture apparatus (1×), a part of the evaluations of glucose and lactic acid was "TBD". However, in the simulation period of seven days, both simulation values up to sixth day fell within the range of ±5%×day. As described above, regarding the simulation of culturing Jurkat cells by the first culture apparatus (1×), it was possible to comprehensively grasp the transition of metabolism.

For the condition resulting in "TBD", the simulation value can be improved by performing the simulation again while changing the number of prediction days, increasing the frequency of data acquisition, increasing the number of reference data and the like. Hereinafter, a part of the evaluation results illustrated in Fig. 10 will be specifically described.

### [6-1 Example of Cell Culture of Adherent Cells]

The present inventors conducted three cell culture experiments under different culture conditions using the first culture apparatus to generate three specimens (specimen 1, specimen 2, and specimen 3) of the adherent cells. In each of the three cell culture experiments, cell culture was performed for seven days, and the cell count data was periodically measured. Apart from this, the present inventors performed processing of generating the third cell count data 44 under the culture conditions of the specimens 2 and 3 on the basis of the cell count data (= first cell count data 42) obtained by the cell culture of the specimen 1. Furthermore, the third cell count data 44 was evaluated by comparing the third cell count data 44 obtained under the culture condition of the specimen 2 with the cell count data obtained by the cell culture experiment of the specimen 2. Similarly, the third cell count data 44 was evaluated by comparing the third cell count data 44 obtained under the culture condition of the specimen 3 with the cell count data obtained by the cell culture experiment of the specimen 3.

The conditions under which the three cell culture experiments were carried out are described below.
- HEK293 cells were used as the adherent cells to be cultured.
- Complete medium containing total protein as protein was used.
- After priming the cell culture circuit 16 with PBS, the cell culture circuit 16 was precoated with cellular adhesion factors (fibronectin), and after the precoating, the bag filled with the complete medium was connected to the first supply flow path 162a.
- Gas conditioning was performed, and cells were seeded in the first circulation flow path 162b.
- Cell culture was performed mainly in the second culture form.
- During the cell culture period, the flow rate of the first circulation flow path 162b was set to 20 mL/min, and the flow rate of the second circulation flow path 164b was set to 300 mL/min.

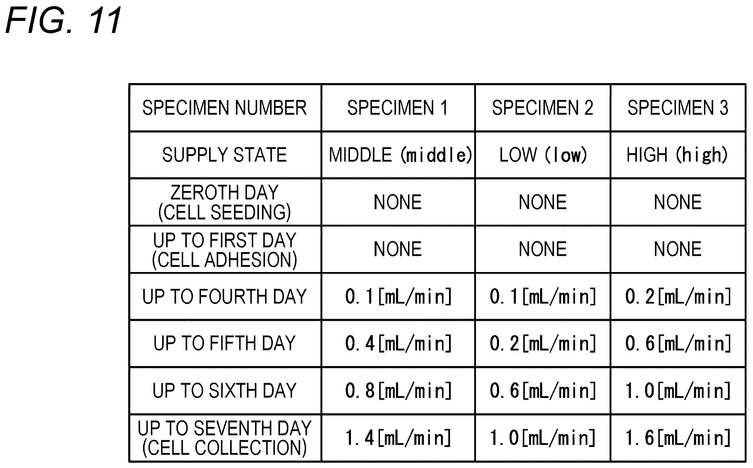

Fig. 11 is a table illustrating the culture condition of each of the three specimens. Fig. 11 illustrates the flow rate of the complete medium supplied from the first supply unit 161 to the first circuit 162 (first supply flow path 162a) for each day.

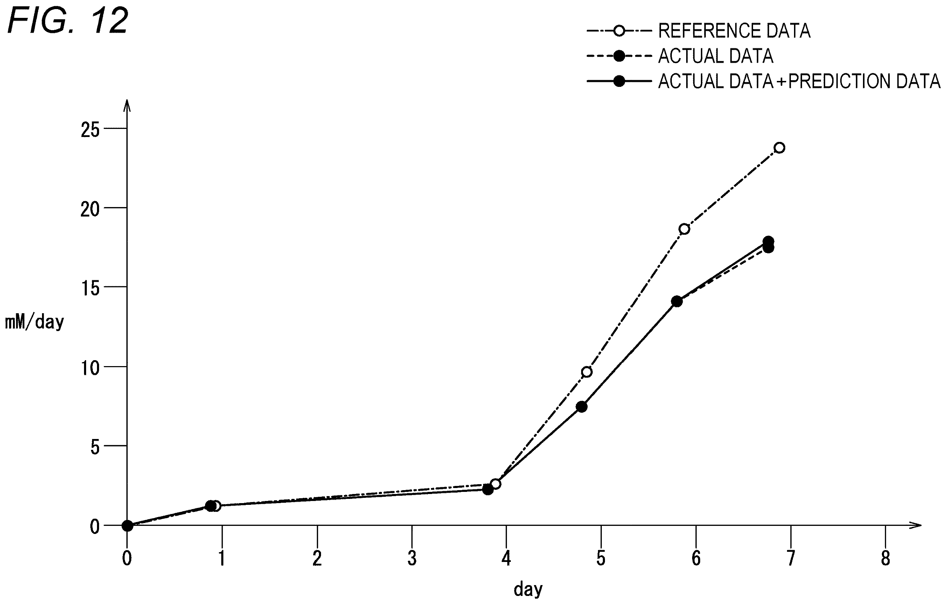

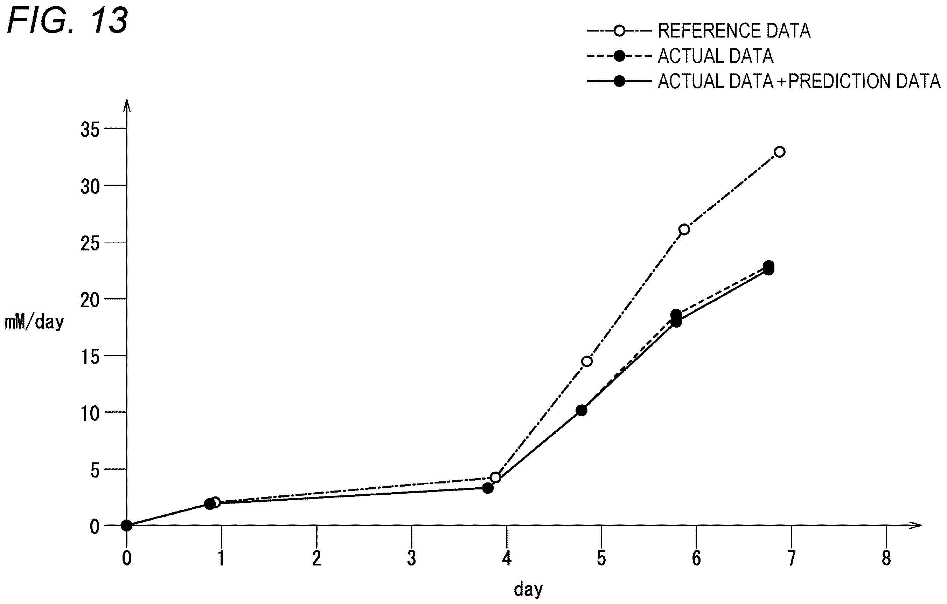

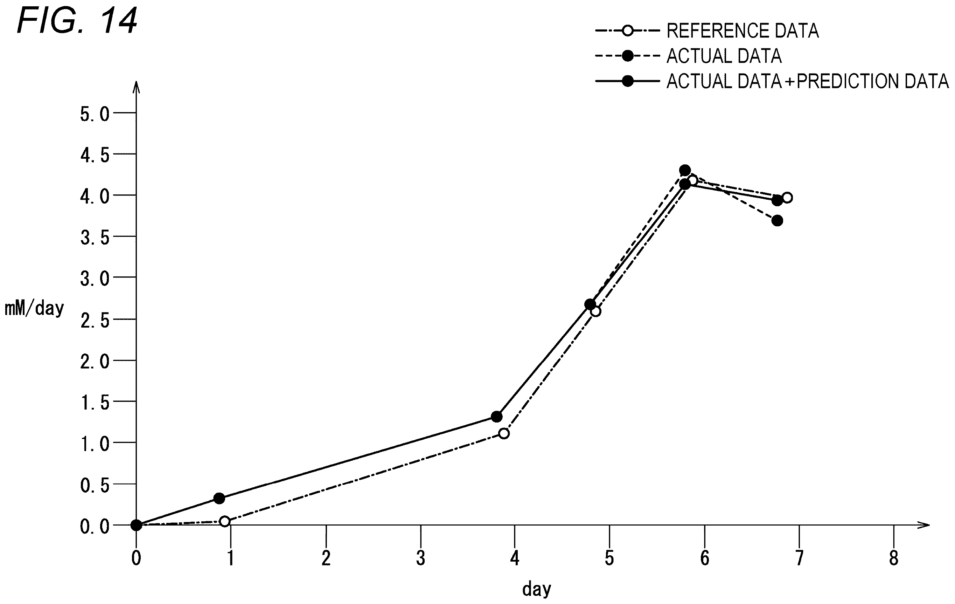

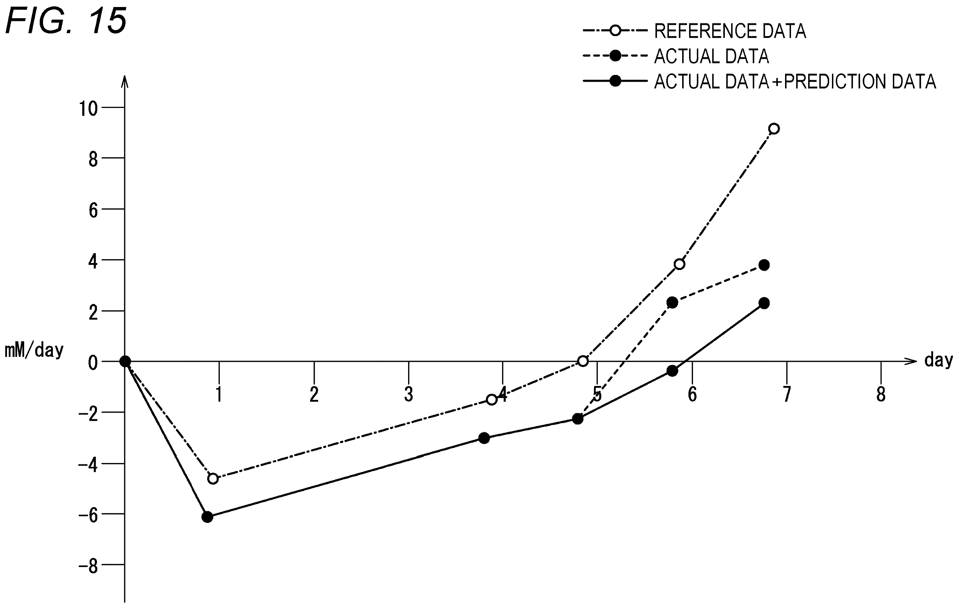

Fig. 12 is a graph illustrating the cell count data (glucose) obtained by simulating the specimen 2 (low) using the specimen 1 (middle) as the reference data. Fig. 13 is a graph illustrating the cell count data (lactic acid) obtained by simulating the specimen 2 (low) using the specimen 1 (middle) as the reference data. Fig. 14 is a graph illustrating the cell count data (oxygen) obtained by simulating the specimen 2 (low) using the specimen 1 (middle) as the reference data. Fig. 15 is a graph illustrating the cell count data (carbon dioxide) obtained by simulating the specimen 2 (low) using the specimen 1 (middle) as the reference data.

In each of Figs. 12 to 15, the graph indicated by a dash-dot line is the reference data. The reference data is the cell count data (= first cell count data 42) obtained by the cell culture of the specimen 1. In each of Figs. 12 to 15, the graph indicated by a broken line is actual data. The actual data is the cell count data obtained by the cell culture of the specimen 2. In each of Figs. 12 to 15, the graph indicated by a solid line is data (= third cell count data 44) obtained by combining the actual data and the prediction data 48.

In each of Figs. 12 to 15, the graph indicated by the solid line substantially fell within ±5%×day of the graph indicated by the broken line. Therefore, the present inventors concluded that the third cell count data 44 was appropriately generated regarding the specimen 2 of the adherent cells.

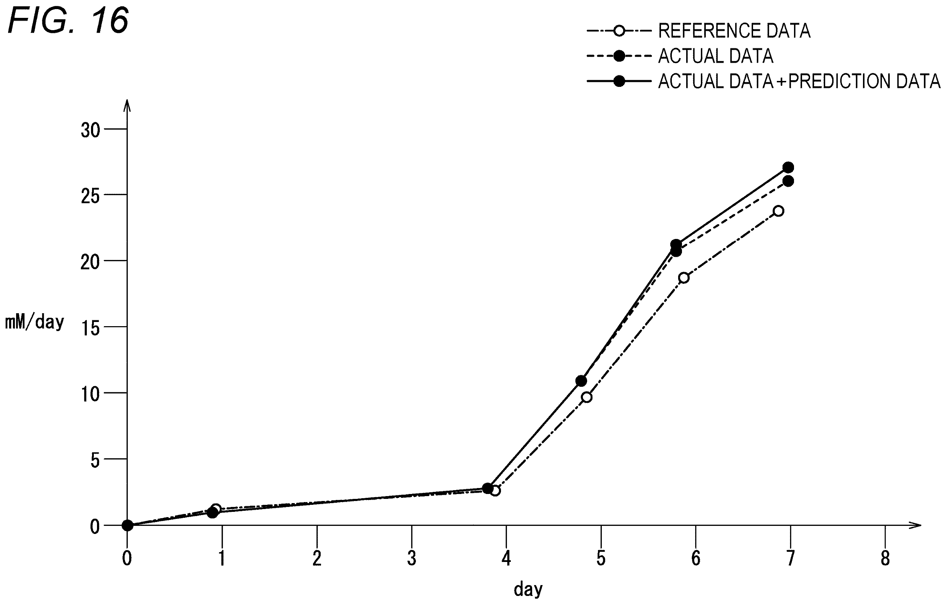

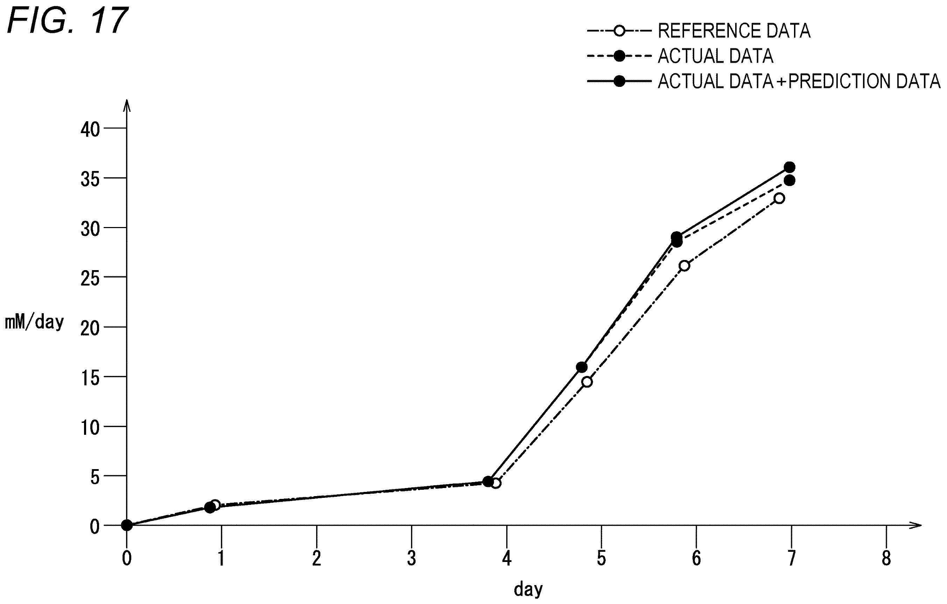

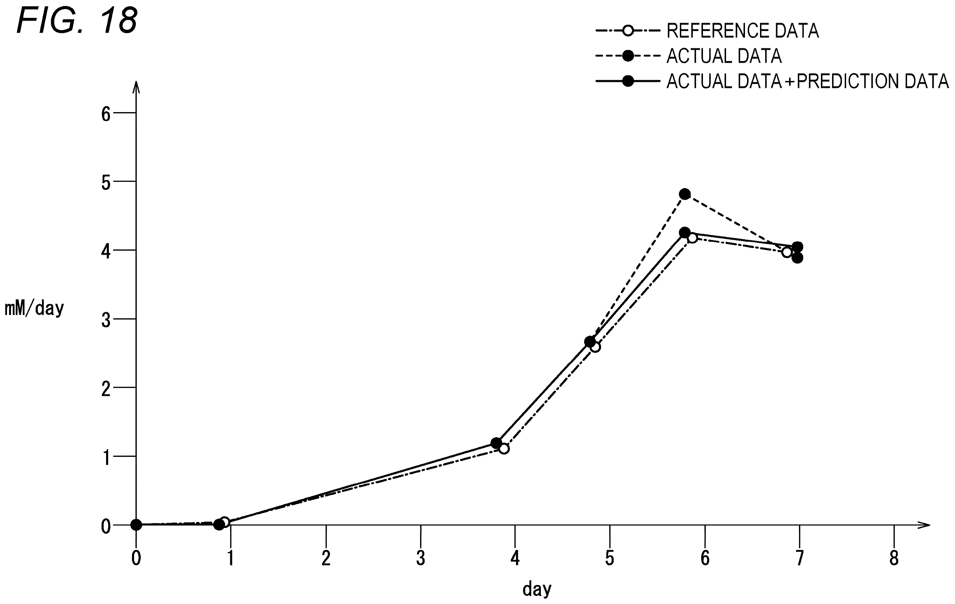

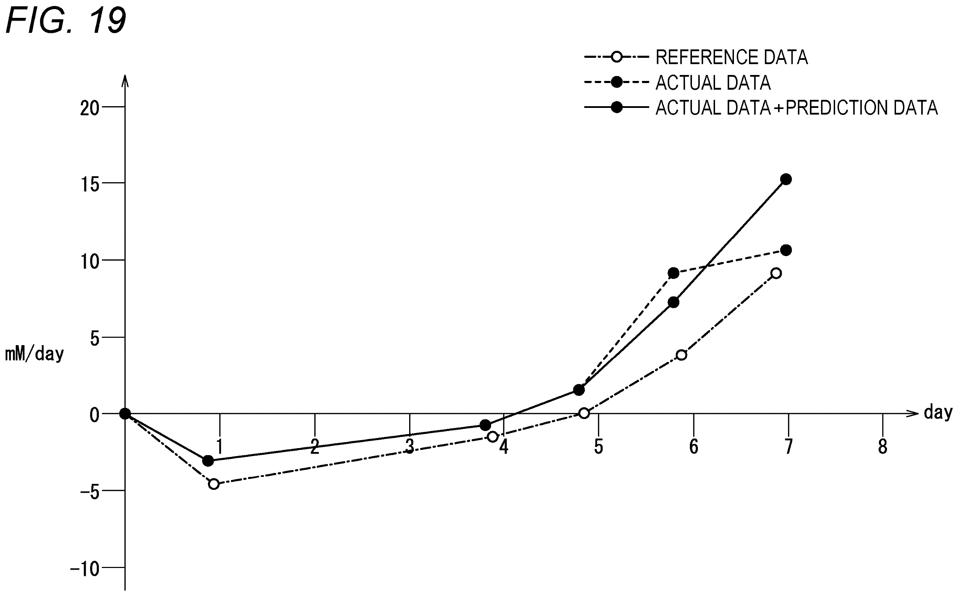

Fig. 16 is a graph illustrating the cell count data (glucose) obtained by simulating the specimen 3 (high) using the specimen 1 (middle) of the adherent cells as the reference data. Fig. 17 is a graph illustrating the cell count data (lactic acid) obtained by simulating the specimen 3 (high) using the specimen 1 (middle) of the adherent cells as the reference data. Fig. 18 is a graph illustrating the cell count data (oxygen) obtained by simulating the specimen 3 (high) using the specimen 1 (middle) of the adherent cells as the reference data. Fig. 19 is a graph illustrating the cell count data (carbon dioxide) obtained by simulating the specimen 3 (high) using the specimen 1 (middle) of the adherent cells as the reference data.

In each of Figs. 16 to 19, the graph indicated by a dash-dot line is the reference data. The reference data is the cell count data (= first cell count data 42) obtained by the cell culture of the specimen 1. In each of Figs. 16 to 19, the graph indicated by a broken line is actual data. The actual data is the cell count data obtained by the cell culture of the specimen 3. In each of Figs. 16 to 19, the graph indicated by a solid line is data (= third cell count data 44) obtained by combining the actual data and the prediction data 48.

In each of Figs. 16 to 19, the graph indicated by the solid line substantially fell within ±5%×day of the graph indicated by the broken line. Therefore, the present inventors concluded that the third cell count data 44 was appropriately generated regarding the specimen 3 of the adherent cells.

### [6-2 Example of Cell Culture of Floating Cells]

The present inventors conducted three cell culture experiments under different culture conditions using the first culture apparatus to generate three specimens (specimen 1, specimen 2, and specimen 3) of the floating cells. In each of the three cell culture experiments, cell culture was performed for seven days, and the cell count data was periodically measured. Apart from this, the present inventors performed processing of generating the third cell count data 44 under the culture conditions of the specimens 2 and 3 on the basis of the cell count data (= first cell count data 42) obtained by the cell culture of the specimen 1. Furthermore, the third cell count data 44 was evaluated by comparing the third cell count data 44 obtained under the culture condition of the specimen 2 with the cell count data obtained by the cell culture experiment of the specimen 2. Similarly, the third cell count data 44 was evaluated by comparing the third cell count data 44 obtained under the culture condition of the specimen 3 with the cell count data obtained by the cell culture experiment of the specimen 3.

The conditions under which the three cell culture experiments were carried out are described below.
- Jurkat cells were used as the floating cells to be cultured.
- Complete medium containing total protein as protein was used.
- The cell culture circuit 16 was primed with PBS, and after the priming, the bag filled with the complete medium was connected to the first supply flow path 162a.
- Gas conditioning was performed, and cells were seeded in the first circulation flow path 162b.
- In an initial stage of the cell culture period (seven days), a series of processes including the cell culture step, a cell dispersion step, and a cell collection step according to the first culture form were repeatedly performed. At the cell dispersion step, the supply and discard of the culture medium were stopped, and the culture medium was circulated in the first circulation flow path 162b. At the cell collection step, the cells diffused into the first circulation flow path 162b at the cell dispersion step were collected inside the bioreactor 160. Here, the cell collection step was performed according to the fifth culture form.
- In middle to later stages of the cell culture period (seven days), a series of processes including the cell culture step, a cell dispersion step, and a cell collection step according to the third culture form were repeatedly performed. Here, the cell collection step was performed according to the fifth culture form.

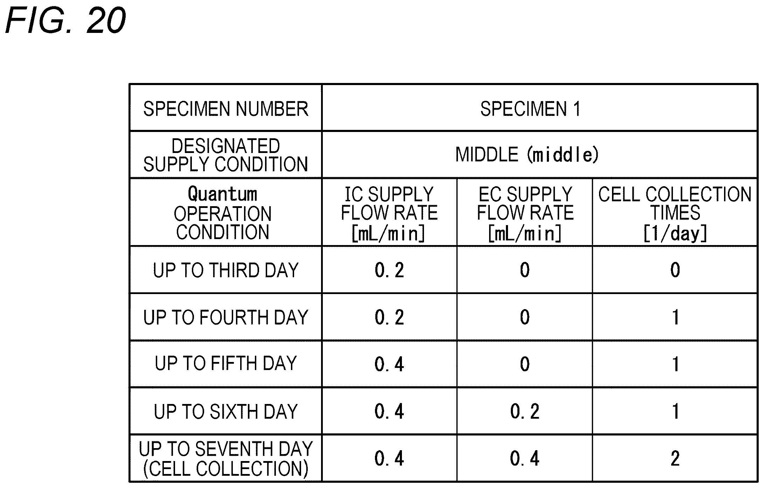

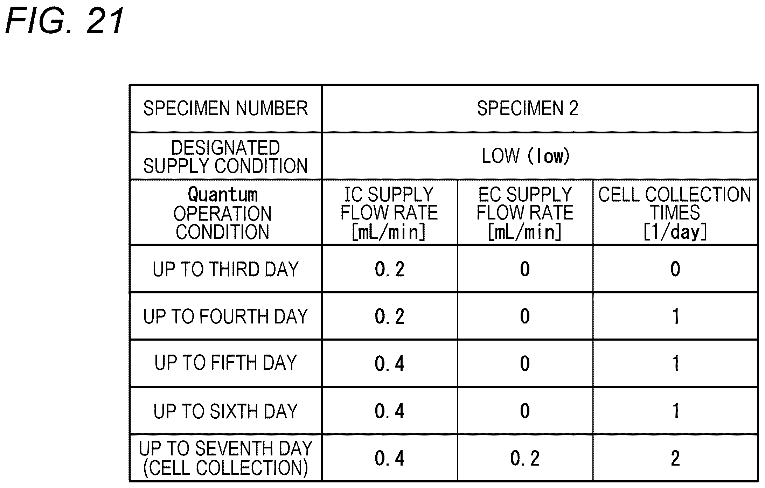

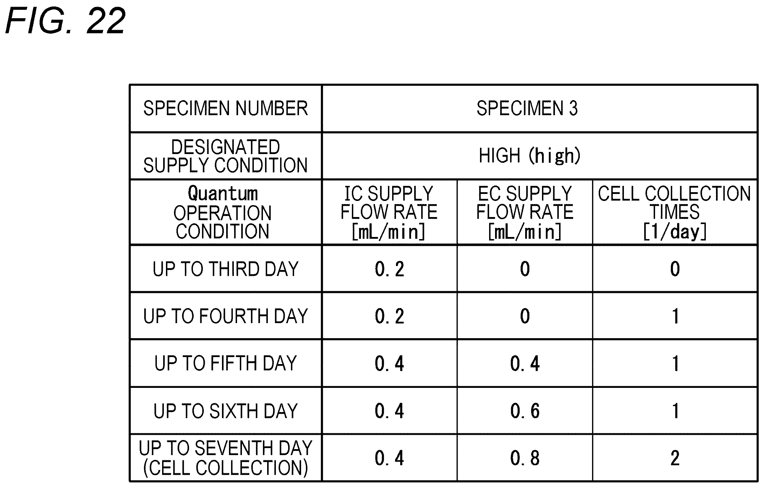

Figs. 20 to 22 are tables illustrating the culture conditions. Fig. 20 illustrates the culture condition of the specimen 1. Fig. 21 illustrates the culture condition of the specimen 2. Fig. 22 illustrates the culture condition of the specimen 3. An "IC supply flow rate" illustrated in each table is the flow rate of the complete medium supplied from the first supply unit 161 to the first circulation flow path 162b via the first supply flow path 162a. An "EC supply flow rate" illustrated in each table is the flow rate of the basal medium supplied from the second supply unit 163 to the second circulation flow path 164b via the second supply flow path 164a.

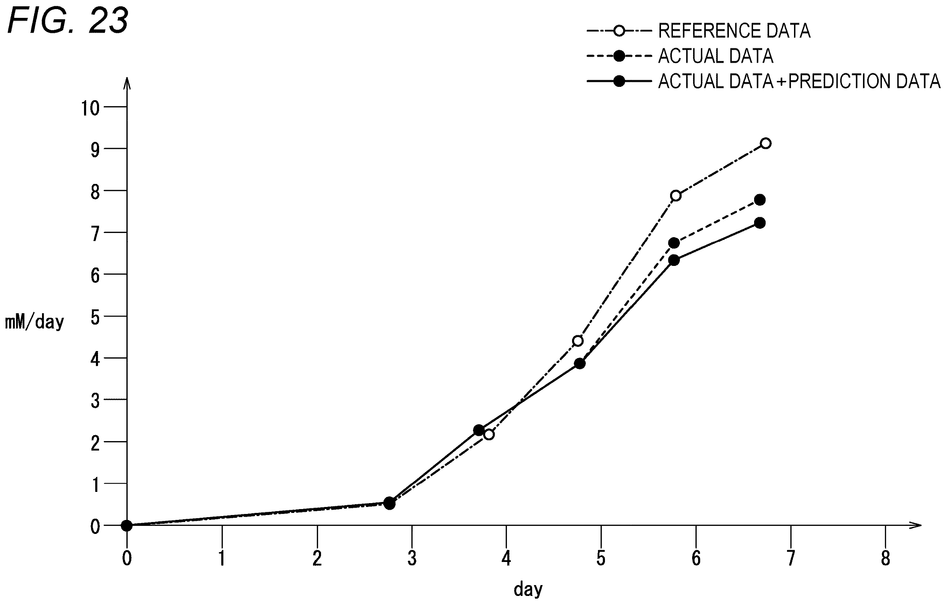

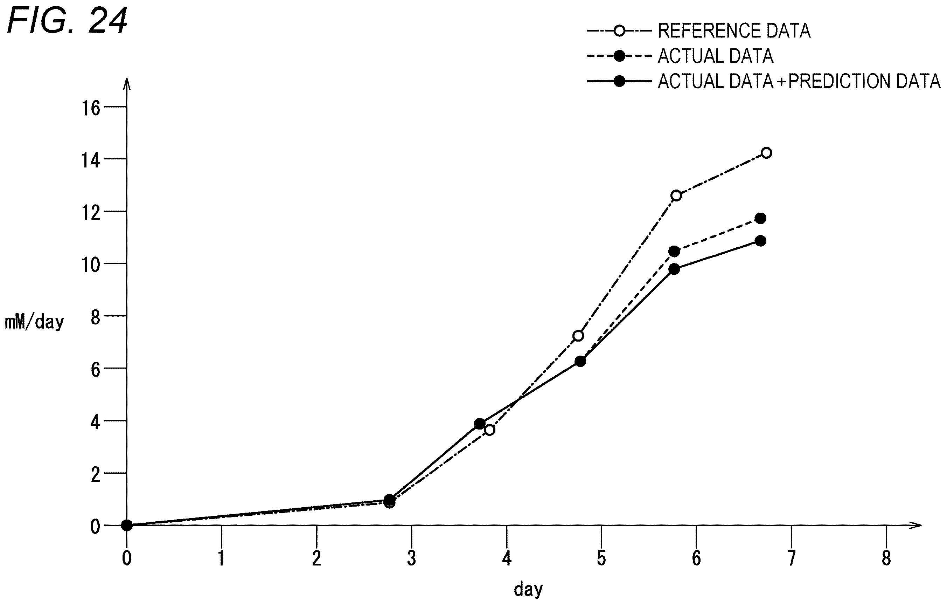

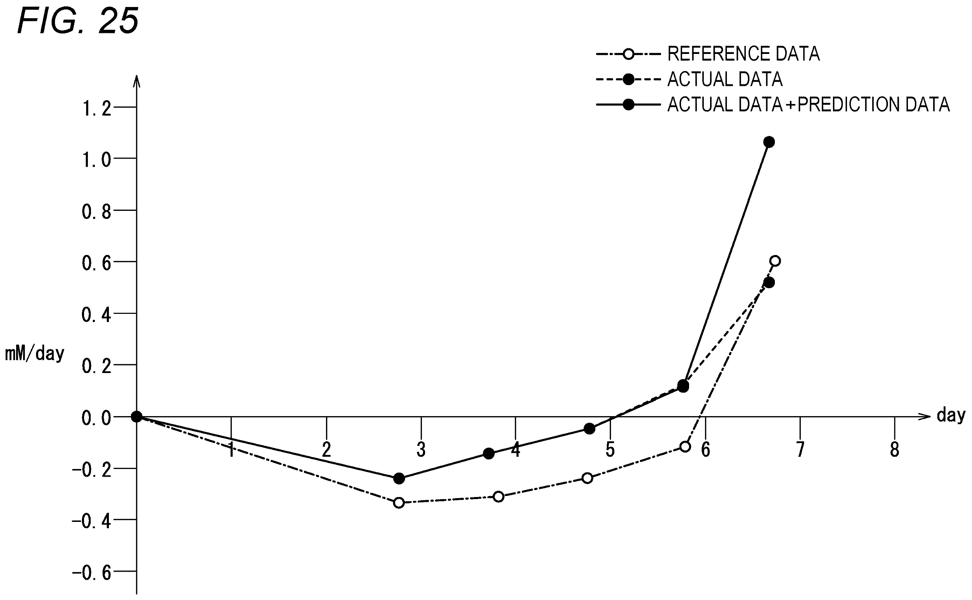

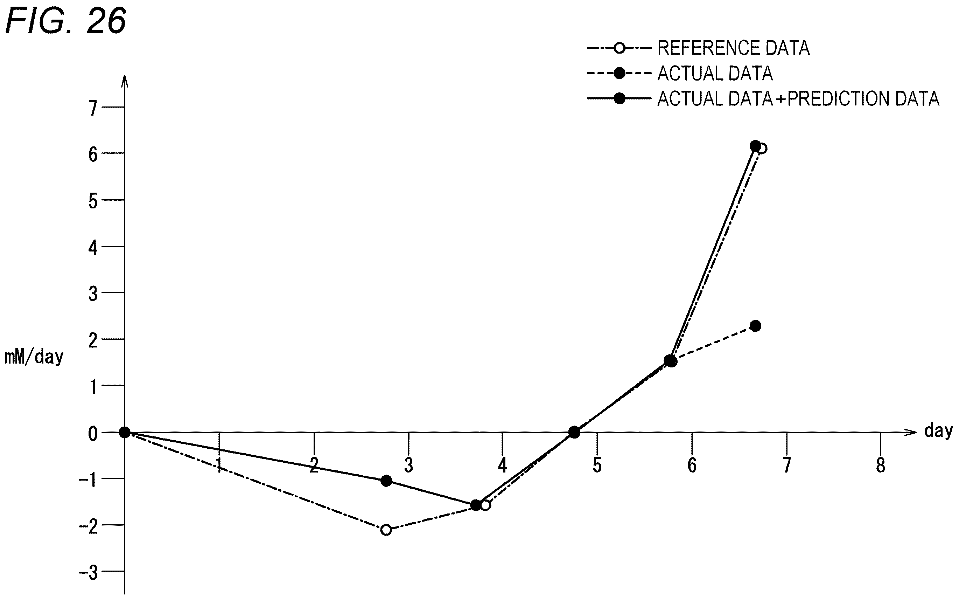

Fig. 23 is a graph illustrating the cell count data (glucose) obtained by simulating the specimen 2 (low) using the specimen 1 (middle) of the floating cells as the reference data. Fig. 24 is a graph illustrating the cell count data (lactic acid) obtained by simulating the specimen 2 (low) using the specimen 1 (middle) of the floating cells as the reference data. Fig. 25 is a graph illustrating the cell count data (oxygen) obtained by simulating the specimen 2 (low) using the specimen 1 (middle) of the floating cells as the reference data. Fig. 26 is a graph illustrating the cell count data (carbon dioxide) obtained by simulating the specimen 2 (low) using the specimen 1 (middle) of the floating cells as the reference data.

In each of Figs. 23 to 26, the graph indicated by a dash-dot line is the reference data. The reference data is the cell count data (= first cell count data 42) obtained by the cell culture of the specimen 1. In each of Figs. 23 to 26, the graph indicated by a broken line is actual data. The actual data is the cell count data obtained by the cell culture of the specimen 2. In each of Figs. 23 to 26, the graph indicated by a solid line is data (= third cell count data 44) obtained by combining the actual data and the prediction data 48.

In each of Figs. 23 to 26, the graph indicated by the solid line substantially fell within ±5%×day of the graph indicated by the broken line. Therefore, the present inventors concluded that the third cell count data 44 was appropriately generated regarding the specimen 2 of the floating cells.

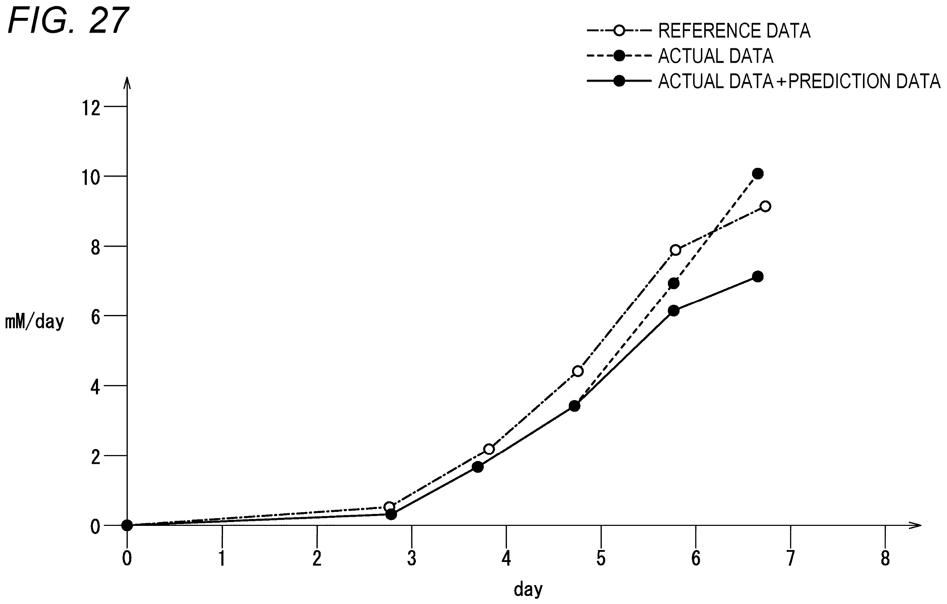

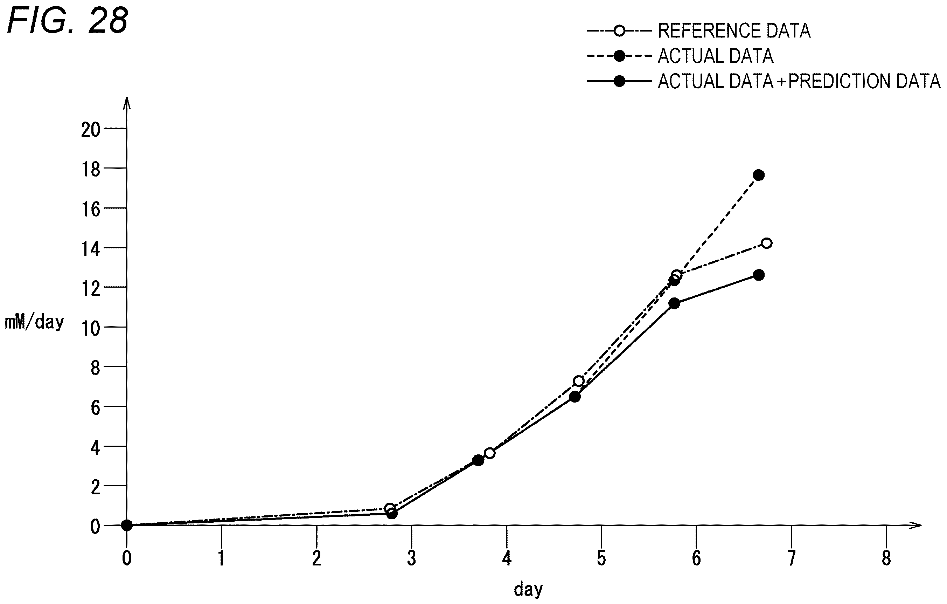

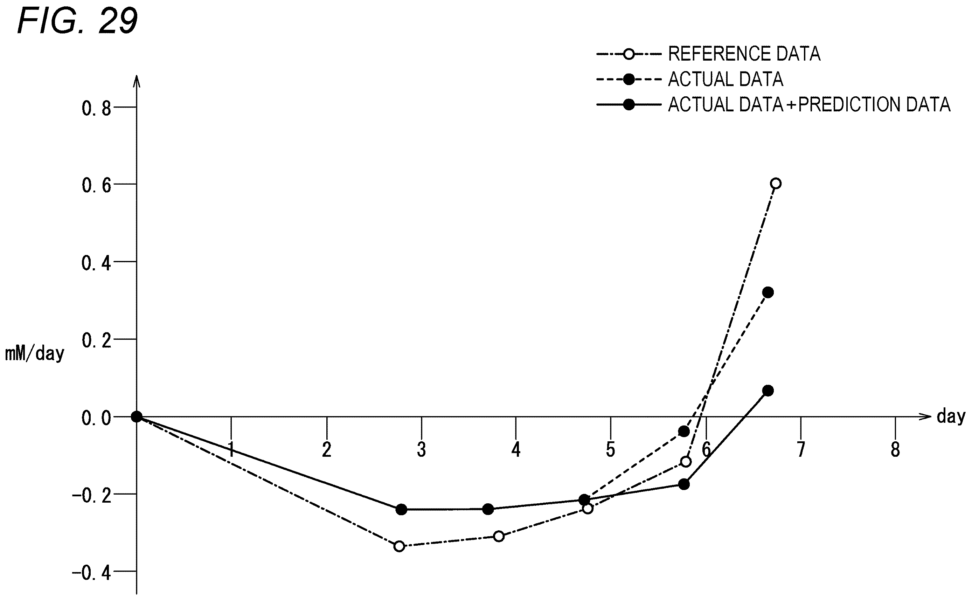

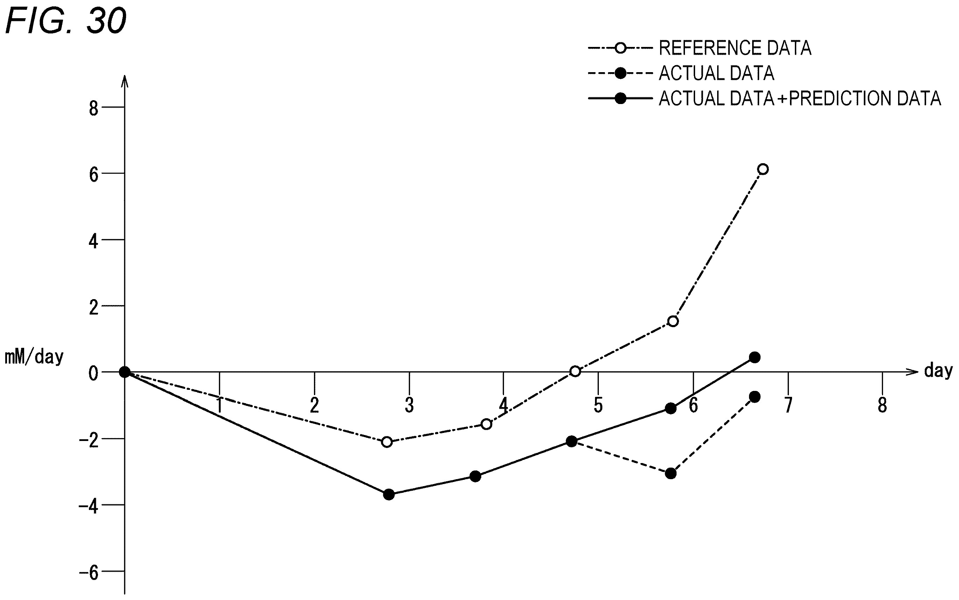

Fig. 27 is a graph illustrating the cell count data (glucose) obtained by simulating the specimen 3 (high) using the specimen 1 (middle) of the floating cells as the reference data. Fig. 28 is a graph illustrating the cell count data (lactic acid) obtained by simulating the specimen 3 (high) using the specimen 1 (middle) of the floating cells as the reference data. Fig. 29 is a graph illustrating the cell count data (oxygen) obtained by simulating the specimen 3 (high) using the specimen 1 (middle) of the floating cells as the reference data. Fig. 30 is a graph illustrating the cell count data (carbon dioxide) obtained by simulating the specimen 3 (high) using the specimen 1 (middle) of the floating cells as the reference data.

In each of Figs. 27 to 30, the graph indicated by a dash-dot line is the reference data. The reference data is the cell count data (= first cell count data 42) obtained by the cell culture of the specimen 1. In each of Figs. 27 to 30, the graph indicated by a broken line is actual data. The actual data is the cell count data obtained by the cell culture of the specimen 3. In each of Figs. 27 to 30, the graph indicated by a solid line is data (= third cell count data 44) obtained by combining the actual data and the prediction data 48.

In each of Figs. 27 to 30, the graph indicated by the solid line substantially fell within ±5%×day of the graph indicated by the broken line. Therefore, the present inventors concluded that the third cell count data 44 was appropriately generated regarding the specimen 3 of the floating cells.

## Claims

1. A data processing apparatus that performs data processing of cell culture performed by a cell culture apparatus including a hollow fiber membrane, the data processing apparatus comprising:
an acquisition unit that acquires first cell count data indicating a change in the number of cells from a start to an end of culture under a first culture condition and second cell count data indicating a change in the number of cells from a start of culture to a present time under a second culture condition;
a calculation unit that calculates a rate ratio that is a ratio between a first change rate that is a change rate of the first cell count data in a first predetermined period and a second change rate that is a change rate of the second cell count data in a second predetermined period corresponding to the first predetermined period; and
a prediction data generation unit that generates prediction data indicating a change in the number of cells after the present time under the second culture condition on a basis of first partial data that is a part after the first predetermined period of the first cell count data and the rate ratio.

2. The data processing apparatus according to claim 1, further comprising:
a display control unit that performs control to display, on a display unit, the number of cells calculated from third cell count data that is data obtained by combining the prediction data with the second cell count data.

3. The data processing apparatus according to claim 1, wherein
the prediction data generation unit generates the prediction data in a case where a deviation amount of the second change rate from the first change rate becomes a predetermined amount or larger.

4. The data processing apparatus according to claim 1, wherein
the first cell count data is data obtained by cell culture performed in the past or data obtained by a simulation of cell culture.

5. The data processing apparatus according to claim 1, wherein
the acquisition unit acquires the first cell count data on a basis of a plurality of pieces of cell count data.

6. A data processing method comprising:
acquiring first cell count data indicating a change in the number of cells from a start to an end of culture under a first culture condition and second cell count data indicating a change in the number of cells from a start of culture to a present time under a second culture condition;
calculating a rate ratio that is a ratio between a first change rate that is a change rate of the first cell count data in a first predetermined period and a second change rate that is a change rate of the second cell count data in a second predetermined period corresponding to the first predetermined period; and
generating prediction data indicating a change in the number of cells after the present time under the second culture condition on a basis of first partial data that is a part after the first predetermined period of the first cell count data and the rate ratio.
